(19) **Europäisches Patentamt**
**European Patent Office**
**Office européen des brevets**

(11) **EP 4 420 609 B1**

(12) **EUROPEAN PATENT SPECIFICATION**

(45) Date of publication and mention
of the grant of the patent:
**25.03.2026 Bulletin 2026/13**

(21) Application number: **22903596.9**

(22) Date of filing: **09.12.2022**

(51) International Patent Classification (IPC):
*A61B 5/369* (2021.01)    *A61B 5/372* (2021.01)
*A61B 5/00* (2006.01)

(52) Cooperative Patent Classification (CPC):
**A61B 5/7264; A61B 5/369; A61B 5/372;**
**A61B 5/7203; A61B 5/7225; A61B 5/7267**

(86) International application number:
**PCT/CN2022/137825**

(87) International publication number:
**WO 2023/104179 (15.06.2023 Gazette 2023/24)**

(54) **TRAINING METHOD AND SYSTEM FOR ELECTROENCEPHALOGRAM SIGNAL NOISE
REDUCTION MODEL FOR ELECTROENCEPHALOGRAM COLLECTION DEVICE**

TRAININGSVERFAHREN UND -SYSTEM FÜR EIN
ELEKTROENZEPHALOGRAMMSIGNALRAUSCHUNTERDRÜCKUNGSMODELL FÜR EINE
ELEKTROENZEPHALOGRAMMSAMMELVORRICHTUNG

PROCÉDÉ ET SYSTÈME D'APPRENTISSAGE POUR UN MODÈLE DE RÉDUCTION DE BRUIT DE
SIGNAL D'ÉLECTRO-ENCÉPHALOGRAMME POUR DISPOSITIF DE RECUEIL
D'ÉLECTRO-ENCÉPHALOGRAMME

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **09.12.2021 CN 202111503309**

(43) Date of publication of application:
**28.08.2024 Bulletin 2024/35**

(73) Proprietor: **Sichuan Neosource Biotektronics
Limited
Chengdu, Sichuan 610041 (CN)**

(72) Inventors:
• **ZHANG, Jiawei**
**Chengdu, Sichuan 610041 (CN)**
• **CHEN, Chao**
**Chengdu, Sichuan 610041 (CN)**
• **PENG, Dingkang**
**Chengdu, Sichuan 610041 (CN)**
• **SHAO, Yongqi**
**Chengdu, Sichuan 610041 (CN)**

• **LI, Guiting**
**Chengdu, Sichuan 610041 (CN)**

(74) Representative: **Wang, Bo**
**Panovision IP**
**Ebersberger Straße 3**
**85570 Markt Schwaben (DE)**

(56) References cited:
**WO-A1-2020/128134    CN-A- 110 531 861
CN-A- 111 598 805    CN-A- 112 545 532
CN-A- 113 081 002    CN-A- 113 349 800
CN-A- 113 412 491    CN-A- 114 190 953
US-B1- 6 876 966**

• **HUANG XUANXI, ZHANG FANGJIAO, ZHANG
JIANYI, YANG TAO: "Application of Generative
Adversarial Network in Medical Image
Generation", JOURNAL OF BEIJING
ELECTRONIC SCIENCE AND TECHNOLOGY
INSTITUTE., vol. 28, no. 4, 1 December 2020
(2020-12-01), pages 36 - 48, XP093069316**

## Description

### CROSS-REFERENCE TO RELATED APPLICATION

This application claims priority to Chinese application No. 202111503309.9, filed on December 9, 2021.

### TECHNICAL FIELD

The present disclosure relates to a field of electroencephalography (EEG) signal processing, and in particular to, a method and system for training an EEG signal denoising model of an EEG collection device.

### BACKGROUND

An EEG signal (e.g., an electroencephalogram) refers to a signal generated by amplifying and recording weak bioelectricity of a human brain, which may be collected by an EEG collection device. The EEG signal is widely used in research in directions of psychology, neuroscience, psychiatry, and brain-computer interfaces.

The EEG signal has features of diversity, nonlinearity, and weakness, and frequency bands of the EEG signal are mainly in low-frequency and ultra-low-frequency ranges. For example, a main frequency of the EEG signal is in a range between 0.5 and 100 Hz, and a signal amplitude of the EEG signal is in a range of 5 and 300 $\mu$V. As the EEG signal is quite weak, it is easily interfered with and drowned out by noise, such as the noise brought by the EEG collection device itself during the collection process, as well as other noises, such as environmental noise, an artifact, etc. So, in order to collect a clean EEG signal, it is necessary to denoise a brainwave signal collected by the EEG collection device, in particular to remove the noise that is brought by the EEG collection device itself during the collection process.

Therefore, there is a need for a method and system for training an EEG signal denoising model of the EEG collection device, to train and obtain a denoising model of the EEG signal for removing the noise in the EEG signal collected by the EEG collection device, especially the noise brought by the EEG collection device itself during the collection process.

In the field of EEG signal processing, CN 113349800 A discloses a technique for training a denoising model using GAN-generated simulated EEG signals combined with noisy real signals to improve robustness across different collection devices and environments. WO 2020/128134 A1 presents a machine learning approach for general data denoising through adversarial training involving a discriminator to classify and refine processed outputs, which can be applied to signal denoising including EEG applications.

## SUMMARY

The invention is set out in the appended claims. One of the embodiments of the present disclosure provides a method implemented on a computing device. The computing device includes at least one processor and at least one storage medium storing a training instruction set for training an EEG signal denoising model of an EEG collection device. The method includes generating an analog EEG signal; obtaining a collected EEG signal by collecting the analog EEG signal through the EEG collection device, the collected EEG signal containing a noise caused by the EEG collection device; and obtaining the EEG signal denoising model of the EEG collection device by training a denoising model based on the collected EEG signal. The training of the denoising model includes obtaining a denoised EEG signal by processing the collected EEG signal through the denoising model; obtaining a discrimination result by processing the denoised EEG signal through a discriminant model, the discrimination result reflecting a probability that the discriminant model determines that the processed denoised EEG signal is a clean EEG signal; and adjusting a model parameter of the denoising model based on the discrimination result.

One of the embodiments of the present disclosure provides a system including at least one storage medium storing a training instruction set for an EEG signal denoising model of an EEG collection device and at least one processor in communication with the at least one storage medium. When performing the instruction set, the at least one processor is configured to generate an analog EEG signal; obtain a collected EEG signal by collecting the analog EEG signal through the EEG collection device, the collected EEG signal containing a noise caused by the EEG collection device; and obtain the EEG signal denoising model of the EEG collection device by training a denoising model based on the collected EEG signal. The training of the denoising model includes obtaining a denoised EEG signal by processing the collected EEG signal through the denoising model; obtaining a discrimination result by processing the denoised EEG signal through a discriminant model, the discrimination result reflecting a probability that the discriminant model determines that the processed denoised EEG signal is a clean EEG signal; and adjusting a model parameter of the denoising model based on the discrimination result.

One of the embodiments of the present disclosure provides a training device for an EEG signal denoising model of an EEG collection device, including at least one storage medium configured to store computer instructions and at least one processor configured to perform the computer instructions to implement the method of: generating an analog EEG signal; obtaining a collected EEG signal by collecting the analog EEG signal through the EEG collection device, the collected EEG signal containing a noise caused by the EEG collection device; and obtaining the EEG signal denoising model of the EEG collection device by training a denoising model based on the collected EEG signal. The training of the denoising model includes obtaining a denoised EEG signal by processing the collected EEG signal through the denoising model; obtaining a discrimination result by processing the denoised EEG signal through a discriminant model, the discrimination result reflecting a probability that the discriminant model determines that the processed denoised EEG signal is a clean EEG signal; and adjusting a model parameter of the denoising model based on the discrimination result.

One of the embodiments of the present disclosure provides a computer-readable storage medium storing computer instructions. When reading the computer instructions, a computer implements a method including generating an analog EEG signal; obtaining a collected EEG signal by collecting the analog EEG signal through the EEG collection device, the collected EEG signal containing a noise caused by the EEG collection device; and obtaining the EEG signal denoising model of the EEG collection device by training a denoising model based on the collected EEG signal. The training of the denoising model includes obtaining a denoised EEG signal by processing the collected EEG signal through the denoising model; obtaining a discrimination result by processing the denoised EEG signal through a discriminant model, the discrimination result reflecting a probability that the discriminant model determines that the processed denoised EEG signal is a clean EEG signal; and adjusting a model parameter of the denoising model based on the discrimination result.

## BRIEF DESCRIPTION OF THE DRAWINGS

The present disclosure is further illustrated by way of exemplary embodiments, which are described in detail by means of the accompanying drawings. These embodiments are not limiting, and in these embodiments, the same numbering denotes the same structure, wherein:

FIG. 1 is a schematic diagram illustrating an application scenario of a method for training an EEG signal denoising model of an EEG collection device according to some embodiments of the present disclosure;
FIG. 2 is a schematic diagram illustrating hardware and/or software assemblies of an exemplary computing device according to some embodiments of the present disclosure;
FIG. 3 is a schematic diagram illustrating hardware and/or software assemblies of an exemplary mobile device according to some embodiments of the present disclosure;
FIG. 4 is a flowchart illustrating an exemplary method for training an EEG signal denoising model of an EEG collection device according to some embodiments of the present disclosure;

FIG. 5 is a flowchart illustrating training of a denoising model of an EEG signal denoising model of an EEG collection device according to some embodiments of the present disclosure;
FIG. 6 is a schematic diagram illustrating a method for training a denoising model in a staged manner according to some embodiments of the present disclosure;
FIG. 7 is a flowchart illustrating an exemplary process for adjusting a model parameter of a denoising model according to some embodiments of the present disclosure; and
FIG. 8 is a module diagram illustrating a system for training an EEG signal denoising model of an EEG collection device according to some embodiments of the present disclosure.

## DETAILED DESCRIPTION

In order to more clearly illustrate the technical solutions of the embodiments of the present disclosure, the accompanying drawings required to be used in the description of the embodiments are briefly described below. Obviously, the accompanying drawings in the following description are only some examples or embodiments of the present disclosure, and it is possible for those skilled in the art to apply the present disclosure to other similar scenarios in accordance with these drawings without creative labor. Unless obviously obtained from the context or the context illustrates otherwise, the same numeral in the drawings refers to the same structure or step.

It may be understood that the terms "system," "device," "unit," and/or "module" as used herein are a way to distinguish between different components, elements, parts, sections, or assemblies at different levels. However, the words may be replaced by other expressions if other words accomplish the same purpose.

As shown in the present disclosure and the claims, unless the context clearly suggests an exception, the words "a," "one," "an," and/or "the" do not refer specifically to the singular, but may also include the plural. Generally, the terms "including" and "comprising" suggest only the inclusion of clearly identified steps and elements. In general, the terms "including" and "comprising" only suggest the inclusion of explicitly identified steps and elements that do not constitute an exclusive list, and the method or apparatus may also include other steps or elements.

Flowcharts are used in the present disclosure to illustrate steps performed by a system in accordance with embodiments of the present disclosure. It may be appreciated that the preceding or following steps are not necessarily performed in an exact sequence. Instead, the steps may be processed in reverse order or simultaneously. Also, it may be possible to add other steps to these processes or remove a step or steps from them.

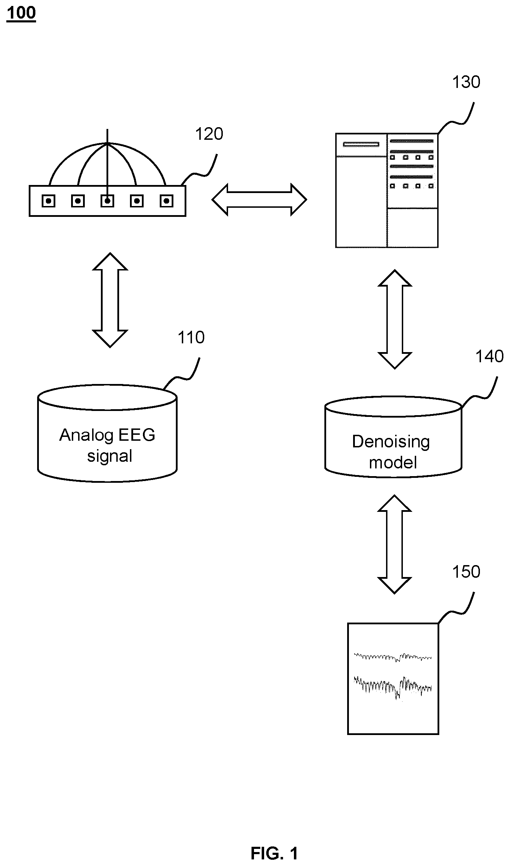

FIG. 1 is a schematic diagram illustrating an application scenario of a method for training an EEG signal denoising model of an EEG collection device according to some embodiments of the present disclosure.

The method for training the EEG signal denoising model of the EEG collection device may be used for training a denoising model to obtain the EEG signal denoising model capable of performing denoising on an EEG signal collected by the EEG collection device. As shown in FIG. 1, an application scenario 100 of the method for training the EEG signal denoising model of the EEG collection device may include an analog EEG signal 110 generated by an EEG signal generator, an EEG collection device 120, and a processing device 130.

The EEG signal generator may include a variety of signal generators capable of generating the analog EEG signal. In some embodiments, the EEG signal generator may include a multi-channel EEG signal generator to simulate a multi-channel EEG signal collected from different brain portions.

The EEG collection device 120 may obtain a collected EEG signal by collecting the analog EEG signal. The EEG collection device 120 may be connected to the processing device 130 (e.g., via a network connection or a direct connection) to enable the processing device 130 to access and process the collected EEG signal. The EEG collection device 120 refers to a device for collecting the EEG signal. For example, the EEG collection device may include an EEG meter, an EEG monitor, a portable EEG collection device, etc. The EEG collection device 120 may include a plurality of electrode slices used for collecting the EEG signal. For example, the electrode slices may be connected to a signal output end of the EEG signal generator to collect the analog EEG signal, or be fitted to various portions of a human brain to collect the EEG signal of the human brain.

The processing device 130 may process various data or information, perform calculations, and determine various results. In some embodiments, the processing device 130 may process the collected EEG signal obtained by the EEG collection device 120. For example, the processing device 130 may train a denoising model 140 based on the collected EEG signal of the EEG collection device to obtain a corresponding EEG signal denoising model corresponding to the EEG collection device. The processing device 130 may process data and/or information obtained from other devices or system components. The processor may perform program instructions based on such data, information, and/or processing results to perform one or more of the functions described in the present disclosure. In some embodiments, the processing device 130 may include one or more sub-processing devices (e.g., a single-core processing device, a multi-core processing device, etc.). In some embodiments, the EEG signal denoising model may also be referred to as a denoising model.

The processing device 130 may process the collected EEG signal (e.g., the EEG signal collected on the human brain) obtained by the EEG collection device 120 through an EEG signal denoising model to obtain a corresponding denoised EEG signal 150 (e.g., a clean EEG signal).

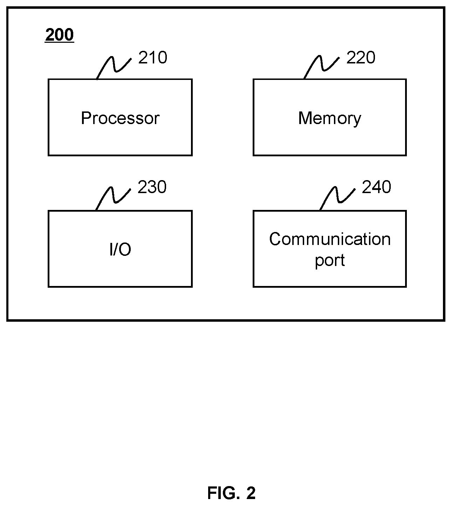

FIG. 2 is a schematic diagram illustrating hardware and/or software assemblies of an exemplary computing device according to some embodiments of the present disclosure. As shown in FIG. 2, a computing device 200 may include a processor 210, a memory 220, an input/output (I/O) 230, and a communication port 240. In some embodiments, the processing device 130 may be implemented on the computing device 200.

The processor 210 may perform computer instructions (program codes). When performing the computer instructions, the processor 210 may cause the processing device 130 to perform the functions of the processing device 130 according to the techniques described herein. The computer instructions may include, for example, routines, programs, objects, components, signals, data structures, processes, modules, and functions that perform particular functions described herein. In some embodiments, the processor 210 may process data and/or images obtained from the EEG collection device 120, the processing device 130, and/or any other components of a system for training the EEG signal denoising model of the EEG collection device. In some embodiments, the processor 210 may include one or more hardware processors, such as a microcontroller, microprocessor, a reduced instruction set computer (RISC), an application specific integrated circuit (ASIC), an application specific instruction set processor (ASIP), a central processing unit (CPU), a graphics processing unit (GPU), a physical processing unit (PPU), a microcontroller unit, a digital signal processor (DSP), a field programmable gate array (FPGA), an advanced RISC machine (ARM), a programmable logic device (PLD), any circuitry or processor capable of performing one or more functions, etc., or any combination thereof.

For illustrative purposes only, only one processor is described in computing device 200. However, it may be noted that the computing device 200 of the present disclosure may also include multiple processors. Accordingly, operations, methods, and/or steps performed by a single processor as described in the present disclosure may also be performed by the multiple processors, either jointly or separately. For example, if both process A and process B are performed in the processor of the computing device 200 of the present disclosure, it may be understood that the process A and the process B may also be performed in the computing device 200 by two or more different processors, either together or separately (e.g., a first processor performs the process A and a second processor performs the process B, or a first processor and a second processor jointly perform the processes A and B).

The memory 220 may store data/information obtained from the EEG collection device 120, the processing device 130, and/or any other components of the system for training the EEG signal denoising model of the EEG collection device. In some embodiments, the memory 220 may include a mass storage device, a removable storage device, a volatile read and write memory, a read-only memory (ROM), etc., or any combination thereof. For example, the mass storage device may include a disk, a CD-ROM, a solid state drive, etc. The removable storage device may include a flash drive, a floppy disk, an optical disk, a memory card, a zip disk, a magnetic tape, etc. The volatile read and write memory may include a random access memory (RAM). The RAM may include a dynamic RAM (DRAM), a double rate synchronous dynamic RAM (DDR SDRAM), a static RAM (SRAM), a thyristor RAM (T-RAM), and a zero capacitance (Z-RAM). The ROM may include a masked ROM (MROM), a programmable ROM (PROM), an erasable programmable ROM (PEROM), an electrically erasable programmable ROM (EEPROM), a compact disk ROM (CD-ROM), and a digital universal disk ROM. In some embodiments, the memory 220 may store one or more programs and/or instructions to perform the exemplary methods described in the present disclosure. For example, the memory 220 may store a program (e.g., in a form of computer-executable instructions) for processing device 130 for determining a calibration model for calibration. As another example, the memory 220 may store a program (e.g., in a form of computer-executable instruction) for the processing device 130 for determining whether the calibration model needs to be updated.

The I/O 230 may input or output a signal, data, and/or information. In some embodiments, the I/O 230 may enable a user to interact with the processing device 130. In some embodiments, the I/O 230 may include an input device and an output device. An exemplary input device may include a keyboard, a mouse, a touch screen, a microphone, etc., or any combination thereof. An exemplary output device may include a display device, a speaker, a printer, a projector, etc., or any combination thereof. An exemplary display device may include a liquid crystal display (LCD), a light emitting diode (LED) based display, a flat panel display, a curved display, a television device, a cathode ray tube (CRT), etc., or any combination thereof.

The communication port 240 may be connected to a network to facilitate data communication. The communication port 240 may establish a connection between the processing device 130 and the EEG collection device 120. The connection may be a wired connection, a wireless connection, or a combination thereof for data transmission and reception. The wired connection may include a cable connection, a fiber optic cable connection, a telephone line connection, etc., or any combination thereof. The wireless connection may include a Bluetooth connection, a Wi-Fi connection, a WiMAX connection, a WLAN connection, a ZigBee connection, a mobile network (e.g., 3G, 4G, 5G, etc.) connection, or a combination thereof. In some embodiments, the communication port 240 may be a standardized port such as RS232, RS485, etc. In some embodiments, the communication port 240 may be a specially designed communication port. For example, the communication port 240 may be designed according to digital imaging and communications in medicine (DICOM) protocol.

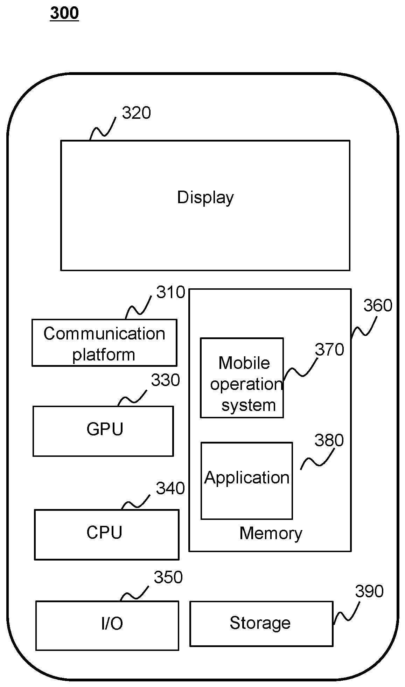

FIG. 3 is a schematic diagram illustrating hardware and/or software assemblies of an exemplary mobile device according to some embodiments of the present disclosure. In some embodiments, the processing device 130 may be implemented on the computing device 200. As shown in FIG. 3, a mobile device 300 may include a communication platform 310, a display 320, a graphics processing unit (GPU) 330, a central processing unit (CPU) 340, an I/O 350, a memory 360, and a storage 390. In some embodiments, any other suitable assemblies, including, but not limited to, a system bus or a controller (not shown), may also be included in the mobile device 300. In some embodiments, a mobile operation system 370 (e.g., iOS, Android, Windows Phone, etc.) and one or more applications 380 may be loaded from the storage 390 into the memory 360 for execution by the CPU 340. The applications 380 may include a browser or any other suitable mobile applications for receiving and rendering information related to image processing or other information from the processing device 130. An interaction between a user and an information flow may be realized through the I/O 350 and provided to, via a network, the processing device 130 and/or other components of the system for training the EEG signal denoising model of the EEG collection device.

To implement the various modules, units, and functions thereof described herein, a computer hardware platform may be used as a hardware platform for one or more of the assemblies described herein. Hardware elements, operating systems, and programming languages for various computers are practically regular and are assumed to be sufficiently familiar to those skilled in the art to make the techniques suitable for generating high-quality images for the scanned objects described herein. The computer containing user interface elements may be used as a personal computer (PC) or other type of workstation or terminal device, or a server when properly programmed. It may be understood that those skilled in the art should be familiar with the structure, programming, and general step of such a computer device, and thus the figures should be self-explanatory thereof.

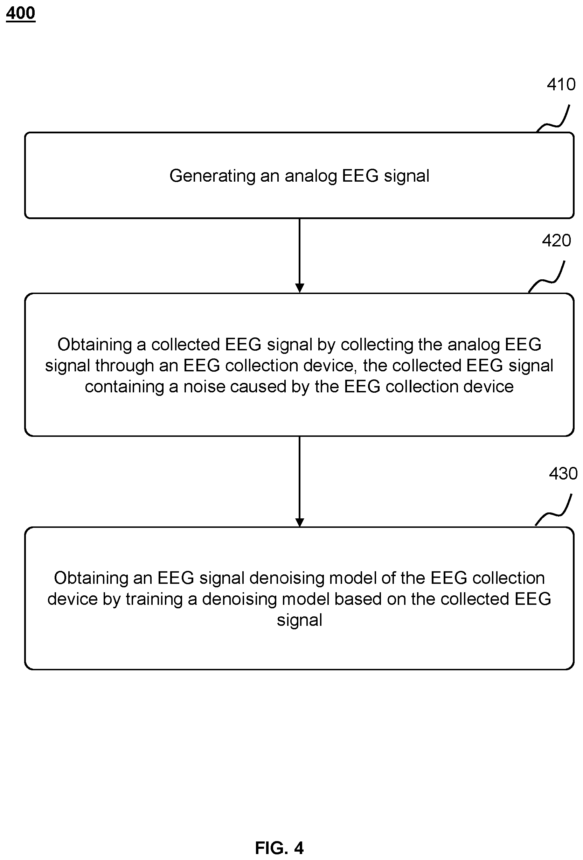

FIG. 4 is a flowchart illustrating an exemplary method for training an EEG signal denoising model of an EEG collection device according to some embodiments of the present disclosure. As shown in FIG. 4, a process 400 includes the following steps.

In step 410, an analog EEG signal is generated. In some embodiments, step 410 may be performed by a generation module 810.

The analog EEG signal refers to a signal that simulates an EEG signal of a human brain. In some embodiments, a processing device may obtain a digital signal by various analog EEG signal generation manners, and then cause an EEG signal generator to convert the digital signal to the analog signal output. For example, the analog EEG signal generation manner may include an EEG signal synthesis algorithm, a generation model for generating the analog EEG signal, a generation network, an encoder, etc.

According to the invention, the analog EEG signal includes a clean analog EEG signal. The analog EEG signal includes a first analog EEG signal reflecting an underlying feature of the EEG signal, and further includes a second analog EEG signal simulating a clean EEG signal. The analog EEG signal further includes an analog EEG signal with noise. Specifically, the analog EEG signal includes a third analog EEG signal with at least one other noise superimposed on the clean EEG signal.

The underlying feature of the EEG signal may include signal underlying features such as an amplitude feature (e.g., an amplitude range), a frequency feature (e.g., a frequency range), etc. The first analog EEG signal may have a wave amplitude that changes regularly and standardly, which is capable of reflecting the underlying features of the EEG signal. For example, the first analog EEG signal may include a sinusoidal signal reflecting the underlying features of the EEG signal.

The second analog EEG signal may be an analog EEG signal that is more complex relative to the first analog EEG signal. A change rule of the second analog EEG signal may be more in line with the change rule of a real EEG signal.

In some embodiments, the second analog EEG signal may be generated by a generation model. In some embodiments, the processing device may input arbitrary data (e.g., random white noise) into the generation model, and the generation model may output the second analog EEG signal.

In some embodiments, the processing device may train the generation model jointly with a discriminant model. The processing device may combine a discriminant model and a generation model to obtain a generative adversarial network (GAN) and train the GAN to obtain the generation model. The generation model may be a generator in the GAN, and the discriminant model may be a discriminator in the GAN. In some embodiments, the generation model may include, but is not limited to, a variety of models such as an NN, an RNN, a CNN, etc. that can generate the analog EEG signal. In some embodiments, the discriminant model may determine whether the input data is the real EEG signal or the analog EEG signal. The discriminant model may include, but is not limited to, a decision tree, an NN, a KNN, etc. that can used to discriminate a type of the input data. In some embodiments, the training of the generation model may include training the generation model based on the discriminant model (which is referred to as a generation model training stage). At this time, the discriminant model may be a trained discriminant model. In the generation model training stage, the processing device may fix a parameter of the discriminant model, input the second analog EEG signal generated by the generation model into the discriminant model, and adjust the parameter of the generation model according to the output of the discriminant model. A training label may be that the discrimination result output by the discrimination model is real, i.e., the discriminant model determines the second analog EEG signal output by the generation model as the real EEG signal.

In some embodiments, the training of the generation model may also include training the discriminant model (which is referred to as the discriminant model training stage). In the discriminant model training stage, the parameter of the generation model may be fixed to train the discriminant model. The processing device may generate a plurality of second analog EEG signals based on the generation model, and select a plurality of real EEG signals from historical data, and then designate the plurality of second analog EEG signals and the plurality of real EEG signals as a training sample. The data types corresponding to the second analog EEG signals and the real EEG signals as the labels to train the discriminant model. As a result, the discriminant model may distinguish between the second analog EEG signals and the real EEG signals as much as possible. In some embodiments, the processing device may alternately perform the discriminant model training stage and the generation model training stage during the training. Through a plurality of iterations of the training, the generation model and the discriminant model may become more and more capable, and when the generation model satisfies conditions such as model convergence, the trained generation model may be obtained. In some embodiments, during the training process of the generation model described above, the data types corresponding to the second analog EEG signal and the real EEG signal in the training samples may be automatically labeled according to the different data sources, or may be manually labeled.

The noise refers to a signal that interferes with the EEG signal. In the present disclosure, other noise refers to the noise other than the noise brought by the EEG signal collection device. The noise may include an artifact signal generated by a blinking of the human body, a heart rate, and a muscle movement of the human body in the human body's EEG signals, and it may also include environmental noise, etc.

In some embodiments, the processing device may generate a third analog EEG signal by superimposing at least one other noise on a clean EEG signal. The processing device may superimpose at least one other noise on a clean real EEG signal (e.g., the denoised EEG signal output from the denoising model after a collected real EEG signal is input into the denoising model) or a clean analog EEG signal (e.g., the second analog EEG signal) to generate the third analog EEG signal. For example, the processing device may superimpose an artificially generated environmental noise signal and the artifact generated by the human heart rate on the clean real EEG signal to generate the third analog EEG signal.

In some embodiments, by generating different third analog EEG signals by superimposing different other noises on the same clean EEG signal, large-scale training data may be obtained based on a small number of clean EEG signals, which solves the problem of small training data for the denoising model.

In step 420, a collected EEG signal is obtained by collecting the analog EEG signal through the EEG collection device, the collected EEG signal containing a noise caused by the EEG collection device. In some embodiments, step 420 may be performed by a collection module 820.

The EEG collection device may collect the EEG signal of the human brain and the analog EEG signal of the EEG signal generator to obtain the corresponding collected EEG signal. For the EEG collection device, as its electronic components need a power supply to operate, situations like a power supply jitter, a temperature drift of the components, a possible tampering of internal lines of each channel, and changes of an external electromagnetic environment usually occur in an operation of the EEG collection device. All of these situations may generate the noise signals during the EEG collection and recording process, which interfere with the EEG signal, e.g., reduce a signal-to-noise ratio of the EEG signal. The noise generated by the operation of the EEG collection device itself is the noise brought about by the EEG collection device. For example, when the third analog EEG signal is the EEG signal with an artificially generated environmental noise superimposed on the clean real EEG signal and the EEG collection device collects the third analog EEG signal, the EEG signal collected by the EEG collection device may have both the original environmental noise and the noise caused by the EEG collection device.

In step 430, an EEG signal denoising model of the EEG collection device is obtained by training a denoising model based on the collected EEG signal. In some embodiments, step 430 may be performed by a training module 830.

The denoising model refers to a model that removes the noise from the input collected EEG signal and outputs a denoised EEG signal (e.g., the clean EEG signal). In some embodiments, the denoising model may include, but is not limited to, various models such as an NN, an RNN, a CNN, etc., that are capable of performing the denoising processing on the EEG signal.

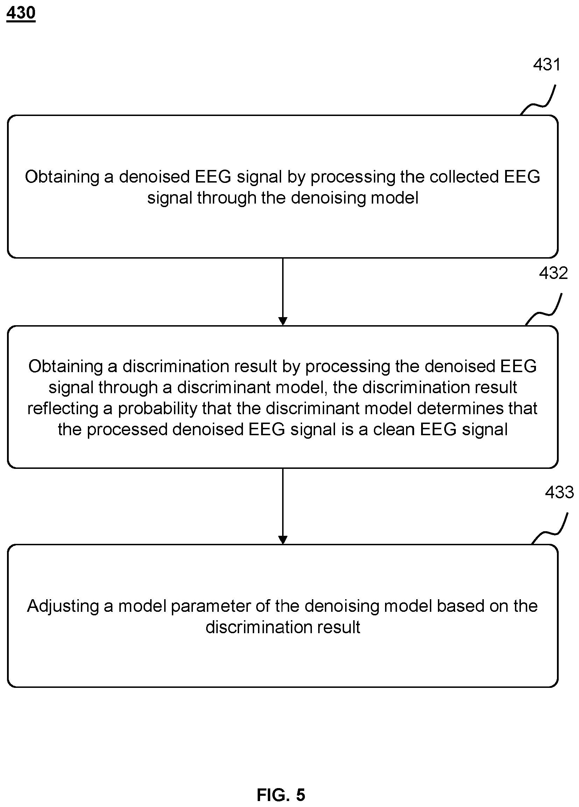

In some embodiments, the processing device may train the denoising model in conjunction with the discriminant model to obtain a desired denoising model of the EEG signal corresponding to the EEG collection device. A main role of the discriminant model is to make the denoised signal output by the denoising model as clean an EEG signal as possible. It may be noted that the discriminant model that is joint during the training of the denoising model and the discriminant model that is joint during the training of the generation model that generates the second analog EEG signal are different models, although their model structures may be the same. Referring to FIG. 5, in some embodiments, the training of the denoising model may include the following steps.

In step 431, a denoised EEG signal is obtained by processing the collected EEG signal through the denoising model.

The processing device may input the collected EEG signal into the denoising model for the denoising processing, and the output of the denoising model may be referred to as the denoised EEG signal.

In step 432, a discrimination result is obtained by processing the denoised EEG signal through a discriminant model, the discrimination result reflecting a probability that the discriminant model determines that the processed denoised EEG signal is a clean EEG signal.

The discrimination result output by the discriminant model may be expressed as a probability or a score. For example, 1 indicates the data being processed (i.e., the input data) (e.g., the denoised EEG signal output by the denoising model) is a clean EEG signal; 0 indicates that the data being processed (i.e., the input data) is not a clean EEG signal, i.e., the input data is the EEG signal containing the noise. The higher the probability or score of the discriminant model output, the higher the probability that the corresponding input data is the clean EEG signal.

In some embodiments, the discriminant model may be a multiclassification model. A plurality of types corresponding to the multiclassification may include the EEG signals with various types of noises (e.g., the EEG signal with eye movement artifacts, the EEG signal with muscle movement artifacts, the EEG signal with environmental noise, etc.) and the clean EEG signal. The discrimination result may also reflect a probability of discriminating the data being processed as the EEG signals with various types of noises. In such cases, the discrimination result may include a probability or score that the denoised EEG signal belongs to each of the plurality of types. For example, the denoised EEG signal obtained by the denoising model may be input into the discriminant model, and the discrimination result obtained by the discriminant model (which is also referred to as a multiclassification result) may include a probability that the denoised EEG signal belongs to a plurality of types. For example, a probability for the denoising EEG signal obtained by the discriminant model to belong to the EEG signal with an eye movement artifact may be 0.2, a probability for the EEG signal to belong to the EEG signal with a muscle movement artifact may be 0.2, a probability for the denoised EEG signal to belong to the EEG signal with the environmental noise may be 0.3, and a probability for the denoised EEG signal to belong to the clean EEG signal may be 0.6. The processing device may take the type with the highest probability as a classification result of the denoised EEG signal.

It can be understood that during the training, the denoising capability of the denoising model changes from weak to strong. When the denoising capability of the denoising model is weak, there may still be the noise in the denoised signal output from the denoising model, so the output of the discriminant model may vary with the capability of the denoising model during the training process.

In step 433, a model parameter of the denoising model is adjusted based on the discrimination result.

When training the denoising model, the processing device may fix the parameter of the discriminant model (in such cases, the discriminant model may be regarded as the trained discriminant model). A target of the adjusting the model parameter of the denoising model by training may include making the denoising model to remove as much noise as possible from the collected EEG signal and output an as clean as possible denoised EEG signal. As a result, after inputting the denoised EEG signal into the discriminant model, the obtained discriminant result may indicate that a probability of the input data being a clean EEG signal is relatively great or is 1.

In some embodiments, as previously described, the discriminant model may be the multiclassification model, and the plurality of types corresponding to the multiclassification may include the EEG signals with noises of various types and the clean EEG signal. The processing device may input the denoised EEG signal obtained by the denoising model into the discriminant model, and the discrimination result obtained by the discriminant model (which is referred to as a multiclassification result) may include a probability that the denoised EEG signal belongs to the plurality of types. The processing device may use a type with the highest probability as the classification result of the denoised EEG signal. When the discriminant model in the training of the denoising model based on the collected EEG signal is the multiclassification model, an optimization target for adjusting the denoising model based on the discrimination result may be to make the classification result of the denoised EEG signal outputted from the denoising model as clean as possible. The processing device may determine a plurality of losses regarding the plurality of types based on the discrimination result, and determine a loss function of the denoising model based on the plurality of losses. In some embodiments, the processing device may determine a weight corresponding to each loss regarding each type, determine a plurality of loss items based on the plurality of losses and the weights corresponding to the plurality of losses, and determine a loss function based on the plurality of loss items, and further adjust the model parameter of the denoising model based on the loss function. The loss regarding a certain type may be determined based on a difference between a probability corresponding to the type output by the discriminant model and a label value. The label value corresponding to the type of the EEG signal with the noises of the various types may be 0. The label value corresponding to the type of the clean EEG signal may be 1.

The target for adjusting the model parameter of the denoising model based on the loss function may include minimizing the value of each loss or loss item, i.e., minimizing the value of the loss or the loss item regarding the type of EEG signal with the noises of various types (e.g., the loss or the loss term regarding a type of EEG signal with various artifacts, the loss or the loss term regarding a type of EEG signal with environmental noise), and minimizing the value of the loss or the loss term regarding the type of clean EEG signal. Through this embodiment, it may be possible to realize a targeted adjustment of the model parameter of the denoising model to specifically improve the ability of the denoising model to remove a certain portion of noise. For example, if the discriminant model outputs a classification result as an EEG signal with eye movement artifacts, the model parameter of the denoising model may be adjusted in a targeted manner to improve the ability of the denoising model to remove the eye movement artifacts.

In some embodiments, the process of training the denoising model may further include training the discriminant model. The training the discriminant model may include inputting a plurality of denoised EEG signals and the clean EEG signal into an initial discriminant model, outputting the discrimination results corresponding to the denoised EEG signals and the clean EEG signal from the initial discriminant model, adjusting the model parameter of the initial discriminant model, so that the initial discriminant model is able to better distinguish between the denoised EEG signals and the clean EEG signal. As a result, the initial discriminant model may better discriminate whether the input signal is the clean EEG signal. In some embodiments, as described previously, the discriminant model may be the multiclassification model. In such cases, the model parameter of the discriminant model may be adjusted to enable the discriminant model to better differentiate between the plurality of types of EEG signal. The plurality of types may include EEG signals with the noises of various types (e.g., the EEG signal with eye movement artifacts, the EEG signal with muscle movement artifacts, the EEG signal with environmental noises, etc.) and the clean EEG signal.

Through multiple iterations of training, when the denoising model satisfies a condition (e.g., the model convergence), the trained denoising model may be obtained, which is the desired EEG signal denoising model corresponding to the EEG collection device. The EEG signal denoising model corresponding to the EEG collection device obtained by training may be used to remove the noise in the collected EEG signal, especially the noise brought by the EEG collection device itself, to obtain the clean EEG signal.

In some examples not according to the invention, the processing device may train the denoising model based on the collected EEG signal corresponding to a kind of the analog EEG signal. For example, the processing device may train the denoising model based on the collected EEG signal corresponding to the second analog EEG signal or the collected EEG signal corresponding to the third analog EEG signal.

According to the invention, the processing device trains the denoising model based on the collected EEG signals corresponding to multiple kinds of analog EEG signals. The processing device trains the denoising model based on the collected EEG signal corresponding to the first analog EEG signal, the collected EEG signal corresponding to the second analog EEG signal, and the collected EEG signal corresponding to the third analog EEG signal.

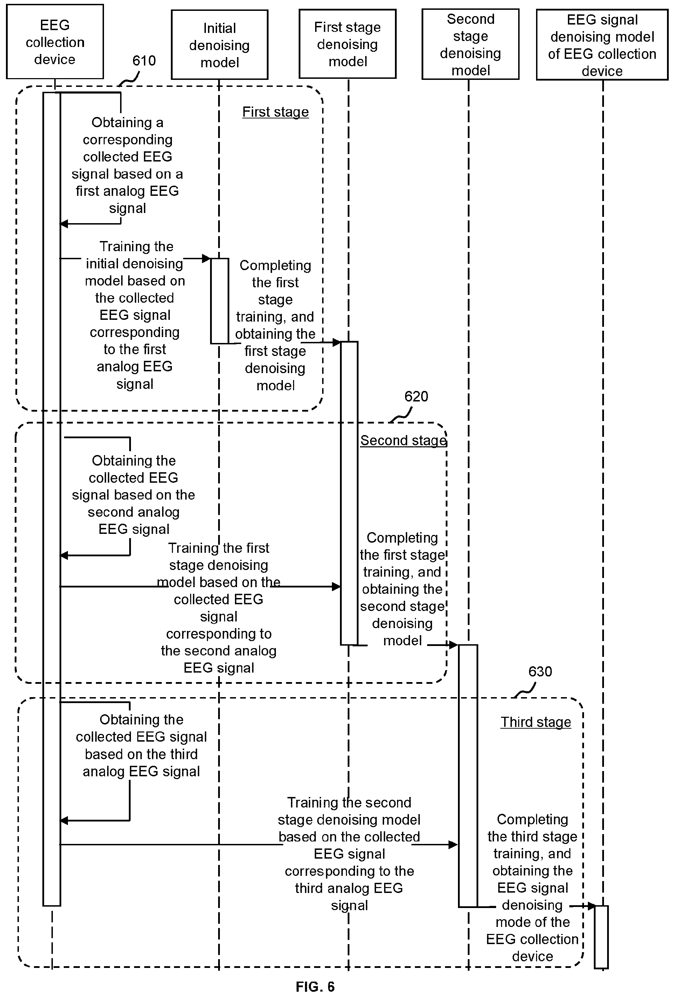

The processing device trains the denoising model in stages. As the training stages progress, the processing device may gradually improve the noise reduction capability of the denoising model in a targeted manner. The processing device trains the denoising model in a plurality of stages based on the collected EEG signals corresponding to a plurality of analog EEG signals. More description regarding training the denoising model in the plurality of stages based on the collected EEG signals corresponding to the plurality of analog EEG signals may refer to FIG. 6 and the related descriptions, which is not be repeated here.

In some embodiments of the present disclosure, by using the EEG collection device to collect the analog EEG signal, the collected EEG signal may be obtained. Then a training on the denoising model may be performed by combining the discriminant model to obtain the denoising model with a strong denoising ability whose denoised EEG signal output is as clean as possible, which is the EEG signal denoising model of the EEG collection device, thereby realizing that the EEG signal denoising model of the EEG collection device obtained by training may remove the noise in the collected EEG signal, in particular, remove the noise brought by the EEG collection device itself.

FIG. 6 is a schematic diagram illustrating a method for training a denoising model in a staged manner according to some embodiments of the present disclosure.

In some embodiments, the processing device may perform a plurality of stages of training on the denoising model based on a collected EEG signal corresponding to a first analog EEG signal, a collected EEG signal corresponding to a second analog EEG signal, and a collected EEG signal corresponding to a third analog EEG signal, thereby obtaining the EEG signal denoising model corresponding to the EEG collection device. As shown in FIG. 6, the training of the denoising model may include a first stage 610, a second stage 620, and a third stage 630.

The first stage 610 of the training of the denoising model may include the following.

The processing device may train an initial denoising model based on the collected EEG signal corresponding to the first analog EEG signal. The initial denoising model refers to an untrained denoising model. After the initial denoising model is trained in the first stage, the denoising model obtained may be referred to as a first stage denoising model. The training of the initial denoising model in the first stage may be performed using the same process as the training of the denoising model described in step 430. More description regarding the training of the denoising model may refer to step 430 and the related descriptions. As mentioned above, the first analog EEG signal refers to a relatively regular or simple analog EEG signal that reflects an underlying feature of the EEG signal. It may be understood that the collected EEG signal corresponding to the first analog EEG signal does not have a high degree of difficulty in denoising, and accordingly, the first stage denoising model obtained by the training based on the collected EEG signal corresponding to the first analog EEG signal is able to denoise the relatively regular or simple collected EEG signal.

The second stage 620 of the training of the denoising model may include the following.

The first stage denoising model obtained in the first stage of training may be further trained based on the collected EEG signal corresponding to the second analog EEG signal. After the first stage denoising model is trained in the second stage, the obtained denoising model may be referred to as a second stage denoising model. The training of the first stage denoising model in the second stage may be performed using the same process as the training of the denoising model described in step 430. More description regarding the training of the denoising model may refer to step 430 and the related descriptions. As mentioned above, relative to the first analog EEG signal, the second analog EEG signal may be more complex and a change rule of the second analog EEG signal may be more in line with the analog EEG signal of the real EEG signal. It may be understood that the collected EEG signal corresponding to the second analog EEG signal is slightly more difficult for denoising than the collected EEG signal corresponding to the first analog EEG signal. The second stage denoising model trained based on the collected EEG signal corresponding to the second analog EEG signal may be able to perform the denoise on the collected EEG signal corresponding to the real EEG signal.

In some embodiments of the present disclosure, by performing the second stage training on the denoising model, the denoising model may learn about the real EEG signal, so that the denoising model may recognize the noise interspersed in the real EEG signal.

The third stage 630 of the training of the denoising model may include the following.

The second stage denoising model obtained in the second stage of training may be further trained based on the collected EEG signal corresponding to the third analog EEG signal. After the second stage denoising model is trained in the third stage, the EEG signal denoising model corresponding to the EEG collection device may be obtained. The training of the second stage denoising model in the third stage may be performed using the same process as the training of the denoising model described in step 430. More description regarding the training of the denoising model may refer to step 430 and the related descriptions. As mentioned above, the third analog EEG signal may be the EEG signal with other noises superimposed on the clean EEG signal, and the collected EEG signal corresponding to the third analog may be closer to the collected EEG signal of the actual situation. The EEG signal denoise model corresponding to the EEG collection device trained based on the collected EEG signal corresponding to the third analog EEG signal may have a stronger capability of denoising the collected EEG signal in the actual situation.

In some embodiments, the initial denoising model may also be referred to as an initial noise reducer. The first stage denoising model may also be referred to as a first stage denoiser, and the second stage noise model may also be referred to as a second stage denoiser.

In some embodiments of the present disclosure, by performing the first stage training on the denoising model, the denoising model may be made to learn a probability distribution of the noise brought by the operation of the EEG collection device itself, so that such noise may be removed by the first stage denoising model. A denoising capability of the second stage denoising model obtained in the second stage training may be stronger than the first stage denoising model, so that the second stage denoising model is able to remove the superimposed noise brought by the EEG collection device itself when collecting the real EEG signal. Through the third stage training, the EEG signal denoising model may be made more capable of denoising the collected EEG signal in real situations. The method of training the EEG signal denoising model by using different training samples in stages may make the EEG signal denoising model focus on how to denoise the EEG signals collected by the EEG collection device and to avoid an overfit of the EEG denoising model.

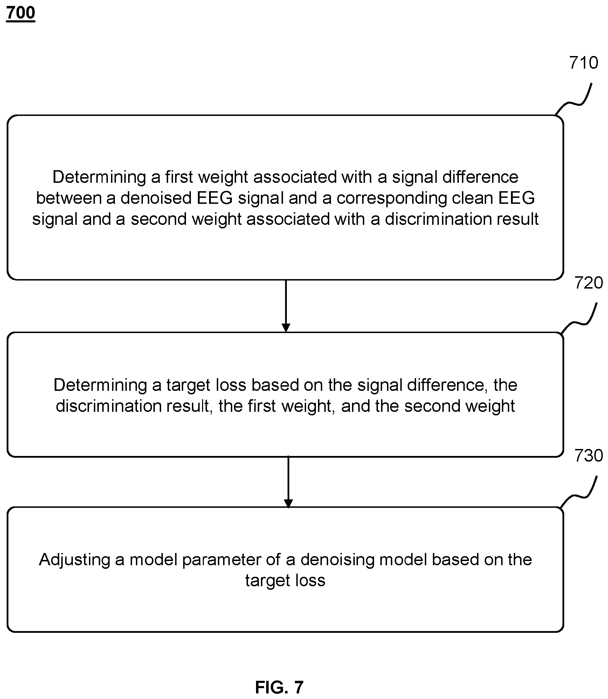

FIG. 7 is a flowchart illustrating an exemplary process for adjusting a model parameter of a denoising model according to some embodiments of the present disclosure. In some embodiments, a process 700 may be performed by a training module.

In some embodiments, each stage of the training of the denoising model may further include determining a signal difference between a denoised EEG signal output by the denoising model and a corresponding clean EEG signal, and adjusting the model parameter of the denoising model based on the signal difference and a discriminant result.

At the first stage 610, the processing device may process the collected EEG signal corresponding to the first analog EEG signal to obtain the corresponding denoised EEG signal based on the initial denoising model. The clean EEG signal corresponding to the denoised EEG signal obtained in the first stage 610 may be the first analog EEG signal. In such cases, the signal difference may be a signal difference between the denoised EEG signal obtained in the first stage 610 and the first analog EEG signal. In the second stage 620, the processing device may process the collected EEG signal corresponding to a second analog EEG signal to obtain the corresponding denoised EEG signal based on the first stage denoising model, and the clean EEG signal corresponding to the denoised EEG signal obtained in the second stage 620 may be the second analog EEG signal. In such cases, the signal difference may be a signal difference between the denoised EEG signal obtained in the second stage 620 and the second analog EEG signal. In the third stage 630, the processing device may process the collected EEG signal corresponding to a third analog EEG signal to obtain the corresponding denoised EEG signal based on the second stage denoising model, and the clean EEG signal corresponding to the denoised EEG signal obtained in the third stage 630 may be the clean EEG signal of the third analog EEG signal before the third analog EEG signal is superimposed with other noises. In such cases, the signal difference may be a signal difference between the denoised EEG signal obtained in the third stage 630 and the clean EEG signal before the third analog EEG signal is superimposed with the other noises.

As shown in FIG.7, the adjusting the parameter of the denoising model based on the signal difference and the discriminant result may specifically include the following steps.

In step 710, a first weight associated with the signal difference between the denoised EEG signal and the corresponding clean EEG signal and a second weight associated with the discrimination result are determined.

The first weight may indicate an important degree of the signal difference for model optimization in the current training stage.

The second weight may indicate an important degree of the discrimination result for the model optimization in the current training stage.

In some embodiments, the first weight and the second weight may be preset. For different stages of the training of the denoising model, magnitudes of the first weight and the second weight may be different. As a result, in different training stages, the importance degrees of the signal differences and the discrimination results for the model optimization may be different.

As mentioned above, with the progression of the training stage, the denoising difficulty of the collected EEG signals may gradually increase, and the denoising ability of the trained denoising model may be gradually improved. Specifically, the ability of the denoising model may be improved from denoising a simple or regular collected EEG signal to denoising a complex collected EEG signal, and may further be improved to denoising the complex collected EEG signals with a variety of different noises. When training the denoising model with the ability to denoise the simple or regular collected EEG signal (i.e., the first training stage), a greater first weight and a smaller second weight may be set to make the model optimization focus more on the signal difference and less on the discrimination result. The denoising model obtained from training (the first stage denoising model) may output a denoised EEG signal that is as close as possible to the clean signal corresponding to the input signal. When the denoising model is trained to have the ability to denoise the complex collected EEG signal (i.e., the second stage), due to the increased complexity of the collected EEG signal, it is hoped that at this point, the denoising model may better learn and remove the distributions of more realistic and complex noises, and may no longer cause the denoising model to output the denoised EEG signal that is as close as possible to the corresponding clean signal. In such cases, it may be possible to reduce the first weight and increase the second weight based on the first stage, so that the second weight may be greater than the first weight, allowing the model optimization to focus more on the discrimination result and less on the signal difference. When the denoising model is trained to have the ability to noise reduction complex collected EEG signals with many different noises (i.e., the third stage), the first weight may be further reduced and the second weight may be further increased based on the second stage due to the further increase in the complexity of the collected EEG signal.

Exemplarily, the first weight and the second weight in the first stage may be preset to 0.6 and 0.4, respectively. The first weight and the second weight of the second stage may be preset to 0.3 and 0.7, respectively. The first weight and the second weight of the third stage may be preset to 0.1 and 0.9, respectively.

In step 720, a target loss is determined based on the signal difference, the discrimination result, the first weight, and the second weight.

In some embodiments, the target loss may be indicated as a joint loss function including a first loss item with respect to the signal difference and a second loss item with respect to the discrimination result. Exemplarily, the joint loss function may be indicated as:

$$Loss_c = X_N * Loss_A + Y_N * Loss_B$$

where $Loss_A$ denotes a first loss function with respect to the signal difference, $Loss_B$ denotes a second loss function with respect to the discrimination result, $Loss_C$ denotes the joint loss function, $X_N$ denotes the first weight corresponding to the Nth stage of training, $Y_N$ denotes the second weight corresponding to the Nth stage of training, $X_N * Loss_A$ denotes the first loss item, and $Y_N * Loss_B$ denotes the second loss item. N may be 1, 2, or 3.

In step 730, the model parameter of the denoising model is adjusted based on the target loss.

In some embodiments, a target of adjusting the model parameter of the denoising model based on the target loss may include minimizing the target loss. Specifically, the processing device may adjust the model parameter of the denoising model so that a value of the aforementioned first loss item may be minimized, and a value of the aforementioned second loss item may be minimized.

Through the method of training the denoising model in stages as described in some embodiments of the present disclosure, the weights corresponding to the signal difference and the discrimination result may be adjusted as the training stages change. For example, as the complexity of the analog EEG signal collected during the training process increases, the processing device may reduce the first weight of the signal difference and increase the second weight of the discrimination result. As a result, the denoising model may learn more about the clean EEG signal in an earlier stage to understand what the clean EEG signal is, and learn more about how to perform the denoising in a later training stage to recognize how to collect the EEG signal for denoising. In this manner, the denoising model may avoid overfitting based on the better learning of the clean EEG signal, and the generalization ability of the denoising model may be improved.

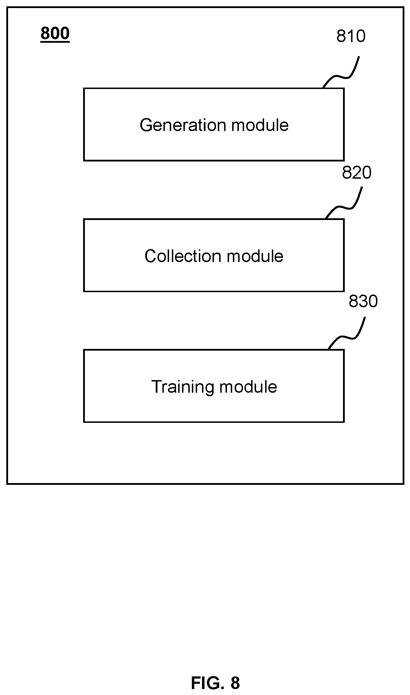

FIG. 8 is a module diagram illustrating a system for training an EEG signal denoising model of an EEG collection device according to some embodiments of the present disclosure.

In some embodiments, a system 800 for training the EEG signal denoising model of the EEG collection device may include a generation module 810, a collection module 820, and a training module 830.

The generation module 810 may be configured to generate an analog EEG signal. More description regarding the analog EEG signal may refer to FIG. 4 and the related descriptions, which are not repeated here. In some embodiments, the generation module 810 may be further configured to obtain a generation model. In some embodiments, the generation module 810 may be further configured to generate a second analog EEG signal based on the generation model. In some embodiments, the generation module 810 may be further configured to obtain the generation model by training a GAN. The generation model may be a generator in the GAN.

The collection module 820 may be configured to obtain a collected EEG signal by collecting the analog EEG signal by the EEG collection device. The collected EEG signal may contain noise caused by the EEG collection device. More description regarding the EEG collection device, the collected EEG signal, and the noise may refer to FIG. 4 and the related descriptions, which are not repeated here.

The training module 830 is configured to obtain an EEG signal denoising model of the EEG collection device by training the denoising model based on the collected EEG signal. The training of the denoising model includes obtaining a denoised EEG signal by processing the collected EEG signal through the denoising model; obtaining a discrimination result by processing the denoised EEG signal through a discriminant model, the discrimination result reflecting a probability that the discriminant model determines that the processed denoised EEG signal is a clean EEG signal; and adjusting a model parameter of the denoising model based on the discrimination result. More description regarding the EEG signal denoising model, the denoised EEG signal, the discriminant model, the discrimination result, and the clean EEG signal may refer to FIG. 4 and the related descriptions, which are not repeated here. As the analog EEG signal includes a first analog EEG signal, a second analog EEG signal, and a third analog EEG signal, the training module 830 is configured to obtain the EEG signal denoising model of the EEG collection device by performing a plurality of stages of training on the denoising model based on the collected EEG signal corresponding to the first analog EEG signal, the collected EEG signal corresponding to the second analog EEG signal, and the collected EEG signal corresponding to the third analog EEG signal, respectively. More description regarding the first analog EEG signal, the second analog EEG signal, and the third analog EEG signal may refer to FIG. 4 and the related descriptions, which are not repeated here. More description regarding the plurality of stages of training may refer to FIG.6 and the related descriptions, which are not repeated here. According to the invention, thetraining module 830 is further configured to obtain a first stage denoising model by performing a first stage training on an initial denoising model based on the collected EEG signal corresponding to the first analog EEG signal, obtain a second denoising model by performing a second stage training on the first stage denoising model based on the collected EEG signal corresponding to the second analog EEG signal, and obtain the EEG signal denoising model of the EEG collection device by perform a third stage training on the second stage denoising model based on the collected EEG signal corresponding to the third analog EEG signal. In some embodiments, the training module 830 may further be configured to determine a first weight associated with a signal difference between the denoised EEG signal and the corresponding clean EEG signal and a second weight associated with the discrimination result; determine a target loss based on the signal difference, the first weight, and the second weight; and adjust the model parameter of the denoising model based on the target loss. More description regarding the signal difference, the first weight, the second weight, and the target loss may refer to FIG. 7 and the related descriptions, which are not repeated here.

It may be understood that the above system and the modules thereof may be implemented in various manners.

It may be noted that the above descriptions of the system for training the EEG signal denoising model of the EEG collection device and the modules thereof are provided only for descriptive convenience, and do not limit the present disclosure to the scope of the embodiments. It may be understood that for those skilled in the art, after understanding the principle of the system, it may be possible to arbitrarily combine the individual modules or form a sub-system to be connected to the other modules without departing from this principle. In some embodiments, the modules in the system for training the EEG signal denoising model of the EEG collection device may be different modules in a single system, or a single module implementing the functions of two or more modules described above. For example, the modules may share a storage module, and the modules may each have a respective storage module. Deformations like these are within the scope of protection of the present disclosure.

Embodiments of the present disclosure further provide a system including at least one storage medium storing a training instruction set for the EEG signal denoising model of the EEG collection device and at least one processor in communication with the at least one storage medium. When performing the instruction set, the at least one processor is configured to generate the analog EEG signal; obtain the collected EEG signal by collecting the analog EEG signal through the EEG collection device, the collected EEG signal containing a noise caused by the EEG collection device; obtain the EEG signal denoising model of the EEG collection device by training a denoising model based on the collected EEG signal. The training of the denoising model may include obtaining a denoised EEG signal by processing the collected EEG signal through the denoising model; obtaining a discrimination result by processing the denoised EEG signal through a discriminant model, the discrimination result reflecting a probability that the discriminant model determines that the processed denoised EEG signal is a clean EEG signal; and adjusting a model parameter of the denoising model based on the discrimination result.

Embodiments of the present disclosure also provide a training device for an EEG signal denoising model of an EEG collection device, including at least one storage medium configured to store computer instructions and at least one processor configured to perform the computer instructions to implement the method of generating an analog EEG signal; obtaining a collected EEG signal by collecting the analog EEG signal through the EEG collection device, the collected EEG signal containing a noise caused by the EEG collection device; and obtaining the EEG signal denoising model of the EEG collection device by training a denoising model based on the collected EEG signal. The training of the denoising model includes obtaining a denoised EEG signal by processing the collected EEG signal through the denoising model; obtaining a discrimination result by processing the denoised EEG signal through a discriminant model, the discrimination result reflecting a probability that the discriminant model determines that the processed denoised EEG signal is a clean EEG signal; and adjusting a model parameter of the denoising model based on the discrimination result.

Embodiments of the present disclosure also provide a computer-readable storage medium storing computer instructions. When reading the computer instructions, a computer implements a method including generating an analog EEG signal; obtaining a collected EEG signal by collecting the analog EEG signal through the EEG collection device, the collected EEG signal containing a noise caused by the EEG collection device; and obtaining the EEG signal denoising model of the EEG collection device by training a denoising model based on the collected EEG signal. The training of the denoising model includes obtaining a denoised EEG signal by processing the collected EEG signal through the denoising model; obtaining a discrimination result by processing the denoised EEG signal through a discriminant model, the discrimination result reflecting a probability that the discriminant model determines that the processed denoised EEG signal is a clean EEG signal; and adjusting a model parameter of the denoising model based on the discrimination result.

The basic concepts have been described above, and it may be apparent to those skilled in the art that the foregoing detailed disclosure serves only as an example and does not constitute a limitation of the present disclosure. The invention is defined by the appended claims.

Also, the present disclosure uses specific words to describe embodiments of the present disclosure. Such as "an embodiment," "one embodiment," and/or "some embodiments" means a feature, structure, or characteristic associated with at least one embodiment of the present disclosure. Accordingly, it may be emphasized and noted that the "one embodiment" or "an embodiment" or "an alternative embodiment" in different places in the present disclosure do not necessarily refer to the same embodiment. In addition, certain features, structures, or characteristics in one or more embodiments of the present disclosure may be suitably combined.

Furthermore, unless expressly stated in the claims, the order of the processing elements and sequences described herein, the use of numerical letters, or the use of other names are not intended to qualify the order of the processes and methods of the present disclosure. While some embodiments of the present disclosure that are currently considered useful are discussed in the foregoing disclosure by way of various examples, it may be appreciated that such details serve only illustrative purposes, and that additional claims are not limited to the disclosed embodiments, rather, the claims are intended to cover all amendments and equivalent combinations that are consistent with the substance and scope of the embodiments of the present disclosure. For example, although the implementation of various components described above may be embodied in a hardware device, it may also be implemented as a software only solution, e.g., an installation on an existing server or mobile device.

Similarly, it may be noted that in order to simplify the presentation of the disclosure of the present disclosure, and thereby aiding in the understanding of one or more embodiments of the present disclosure, the foregoing descriptions of embodiments of the present disclosure sometimes group multiple features together in a single embodiment, accompanying drawings, or in a description thereof. However, this method of disclosure does not imply that the objects of the present disclosure require more features than those mentioned in the claims. Rather, claimed subject matter may lie in less than all features of a single foregoing disclosed embodiment.

Some embodiments use numbers to describe the number of components, attributes, and it should be understood that such numbers used in the description of the embodiments are modified in some embodiments by the modifiers "about," "approximately," or "substantially." Unless otherwise noted, the terms "about," "approximate," or "substantially" indicate that a $\pm 20\%$ variation in the stated number is allowed. Correspondingly, in some embodiments, the numerical parameters used in the present disclosure and the claims are approximations, which vary depending on the desired features of individual embodiments. In some embodiments, the numerical parameters may take into account the specified number of significant digits and employ general place-keeping. While the numerical domains and parameters used to confirm the breadth of their ranges in some embodiments of the present disclosure are approximations, in specific embodiments, such values are set to be as precise as possible within a feasible range.

## Claims

1. A method (400) implemented on a computing device, the computing device including at least one processor and at least one storage medium storing a training instruction set for training an electroencephalogram (EEG) signal denoising model of an EEG collection device, the method comprising:

   generating (410) an analog EEG signal, wherein the analog EEG signal includes a first analog EEG signal reflecting an underlying feature of the analog EEG signal, a second analog EEG signal simulating a clean EEG signal, and a third analog EEG signal with at least one other noise superimposed on the clean EEG signal;
   obtaining (420) a collected EEG signal by collecting the analog EEG signal through the EEG collection device, the collected EEG signal containing a noise caused by the EEG collection device; and
   obtaining (430) the EEG signal denoising model of the EEG collection device by training a denoising model based on the collected EEG signal, the training of the denoising model comprising:

obtaining (431) a denoised EEG signal by processing the collected EEG signal through the denoising model;
obtaining (432) a discrimination result by processing the denoised EEG signal through a discriminant model, the discrimination result reflecting a probability that the discriminant model determines that the processed denoised EEG signal is the clean EEG signal; and
adjusting (433) a model parameter of the denoising model based on the discrimination result, wherein the obtaining the EEG signal denoising model of the EEG collection device by training a denoising model based on the collected EEG signal comprises:

obtaining a first stage (610) denoising model by performing a first stage training on an initial denoising model based on the collected EEG signal corresponding to the first analog EEG signal;
obtaining a second stage (620) denoising model by performing a second stage training on the first stage denoising model based on the collected EEG signal corresponding to the second analog EEG signal; and
obtaining the EEG signal denoising model of the EEG collection device by performing a third stage (630) training on the second stage denoising model based on the collected EEG signal corresponding to the third analog EEG signal.

2. The method of claim 1, the generating an analog EEG signal comprising:

obtaining a generation model; and
generating the second analog EEG signal based on the generation model.

3. The method of in claim 2, the obtaining a generation model comprising:
obtaining the generation model by training a generative adversarial network (GAN), wherein the generation model is a generator in the generative adversarial network.

4. The method of claim 1, the adjusting a model parameter of the denoising model based on the discrimination result comprising:

in current training stage,
determining a first weight associated with a signal difference between the denoised EEG signal and the corresponding clean EEG signal and a second weight associated with the discrimination result;
determining a target loss based on the signal difference, the discrimination result, the first weight, and the second weight; and
adjusting the model parameter of the denoising model based on the target loss.

5. A system, comprising at least one storage medium storing a training instruction set for an EEG signal denoising model (140) of an EEG collection device (120) and at least one processor in communication with the at least one storage medium, wherein when performing the instruction set, the at least one processor is configured to:

generate an analog EEG signal (110), wherein the analog EEG signal includes a first analog EEG signal reflecting an underlying feature of the analog EEG signal, a second analog EEG signal simulating a clean EEG signal, and a third analog EEG signal with at least one other noise superimposed on the clean EEG signal;
obtain a collected EEG signal by collecting the analog EEG signal through the EEG collection device, the collected EEG signal containing a noise caused by the EEG collection device; and
obtain the EEG signal denoising model of the EEG collection device by training a denoising model based on the collected EEG signal, the training of the denoising model comprising:

obtaining a denoised EEG signal (150) by processing the collected EEG signal through the denoising model;
obtaining a discrimination result by processing the denoised EEG signal through a discriminant model, the discrimination result reflecting a probability that the discriminant model determines that the processed denoised EEG signal is the clean EEG signal; and
adjusting a model parameter of the denoising model based on the discrimination result, wherein the obtaining the EEG signal denoising model of the EEG collection device by training a denoising model based on the collected EEG signal comprises:

obtaining a first stage denoising model by performing a first stage training on an initial denoising model based on the collected EEG signal corresponding to the first analog EEG signal;

obtaining a second stage denoising model by performing a second stage training on the first stage denoising model based on the collected EEG signal corresponding to the second analog EEG signal; and

obtaining the EEG signal denoising model of the EEG collection device by performing a third stage training on the second stage denoising model based on the collected EEG signal corresponding to the third analog EEG signal.

6. The system of in claim 5, wherein the at least one processor further is configured to:

obtain a generation model; and
generate the second analog EEG signal based on the generation model.

7. The system of in claim 6, wherein the at least one processor is further configured to:
obtain the generation model by training a generative adversarial network, wherein the generation model is a generator in the generative adversarial network.

8. A training device for an EEG signal denoising model (140) of an EEG collection device (120), including at least one storage medium configured to store computer instructions and at least one processor configured to perform the computer instructions to implement the method of:

generating an analog EEG signal (110), wherein the analog EEG signal includes a first analog EEG signal reflecting an underlying feature of the analog EEG signal, a second analog EEG signal simulating a clean EEG signal, and a third analog EEG signal with at least one other noise superimposed on the clean EEG signal

obtaining a collected EEG signal by collecting the analog EEG signal through the EEG collection device, the collected EEG signal containing a noise caused by the EEG collection device; and

obtaining the EEG signal denoising model of the EEG collection device by training a denoising model based on the collected EEG signal, the training of the denoising model comprising:

obtaining a denoised EEG signal (150) by processing the collected EEG signal through the denoising model;
obtaining a discrimination result by processing the denoised EEG signal through a discriminant model, the discrimination result reflecting a probability that the discriminant model determines that the processed denoised EEG signal is the clean EEG signal; and

adjusting a model parameter of the denoising model based on the discrimination result, wherein the obtaining the EEG signal denoising model of the EEG collection device by training a denoising model based on the collected EEG signal comprises:

obtaining a first stage denoising model by performing a first stage training on an initial denoising model based on the collected EEG signal corresponding to the first analog EEG signal;
obtaining a second stage denoising model by performing a second stage training on the first stage denoising model based on the collected EEG signal corresponding to the second analog EEG signal; and
obtaining the EEG signal denoising model of the EEG collection device by performing a third stage training on the second stage denoising model based on the collected EEG signal corresponding to the third analog EEG signal.

9. A computer-readable storage medium storing computer instructions, wherein when reading the computer instructions, a computer implements a method comprising:

generating an analog EEG signal (110), wherein the analog EEG signal includes a first analog EEG signal reflecting an underlying feature of the analog EEG signal, a second analog EEG signal simulating a clean EEG signal, and a third analog EEG signal with at least one other noise superimposed on the clean EEG signal
obtaining a collected EEG signal by collecting the analog EEG signal through the EEG collection device (120), the collected EEG signal containing a noise caused by the EEG collection device; and
obtaining the EEG signal denoising model (140) of the EEG collection device by training a denoising model based on the collected EEG signal, the training of the denoising model comprising:

obtaining a denoised EEG signal (150) by processing the collected EEG signal through the denoising model;
obtaining a discrimination result by processing the denoised EEG signal through a discriminant model, the discrimination result reflecting a probability that the discriminant model determines that the processed denoised EEG signal is the clean EEG signal; and

adjusting a model parameter of the denoising model based on the discrimination result, wherein the obtaining the EEG signal denoising model of the EEG collection device by training a denoising model based on the collected EEG signal comprises:

obtaining a first stage denoising model by performing a first stage training on an initial denoising model based on the collected EEG signal corresponding to the first analog EEG signal;

obtaining a second stage denoising model by performing a second stage training on the first stage denoising model based on the collected EEG signal corresponding to the second analog EEG signal; and

obtaining the EEG signal denoising model of the EEG collection device by performing a third stage training on the second stage denoising model based on the collected EEG signal corresponding to the third analog EEG signal.

10. The method of claim 1, wherein the first analog EEG signal includes a sinusoidal signal reflecting the underlying feature of the analog EEG signal.

11. The method of claim 4, wherein

the first weight indicates an important degree of the signal difference for model optimization in the current training stage, and the second weight indicates an important degree of the discrimination result for the model optimization in the current training stage.

12. The method of claim 11, further comprising:

in the first training stage, setting the first weight to be greater than the second weight to make the denoising model optimization focus more on the signal difference and less on the discrimination result; and in the second stage, setting the first weight to be smaller than the second weight to make the denoising model optimization focus more on the discrimination result and less on the signal difference.

**Patentansprüche**

1. Verfahren (400), implementiert auf einer Computervorrichtung, wobei die Computervorrichtung mindestens einen Prozessor und mindestens ein Speichermedium einschließt, das einen Trainingsanweisungssatz zum Trainieren eines Elektroenzephalogramm-Signalentrauschungsmodells (EEG-Signalentrauschungsmodells) einer EEG-Erfassungsvorrichtung speichert, wobei das Verfahren Folgendes umfasst:

Erzeugen (410) eines analogen EEG-Signals, wobei das analoge EEG-Signal ein erstes analoges EEG-Signal, das ein zugrunde liegendes Merkmal des analogen EEG-Signals widerspiegelt, ein zweites analoges EEG-Signal, das ein sauberes EEG-Signal simuliert, und ein drittes analoges EEG-Signal mit mindestens einem weiteren Rauschen einschließt, das dem sauberen EEG-Signal überlagert ist; Erhalten (420) eines erfassten EEG-Signals durch Erfassen des analogen EEG-Signals mittels der EEG-Erfassungsvorrichtung, wobei das erfasste EEG-Signal ein durch die EEG-Erfassungsvorrichtung verursachtes Rauschen enthält; und Erhalten (430) des EEG-Signalentrauschungsmodells der EEG-Erfassungsvorrichtung durch Trainieren eines Entrauschungsmodells auf der Grundlage des erfassten EEG-Signals, wobei das Trainieren des Entrauschungsmodells Folgendes umfasst:

Erhalten (431) eines entrauschten EEG-Signals durch Verarbeiten des erfassten EEG-Signals mittels des Entrauschungsmodells; Erhalten (432) eines Unterscheidungsergebnisses durch Verarbeiten des entrauschten EEG-Signals mittels eines Diskriminanzmodells, wobei das Unterscheidungsergebnis eine Wahrscheinlichkeit widerspiegelt, dass das Diskriminanzmodell bestimmt, dass das verarbeitete entrauschte EEG-Signal das saubere EEG-Signal ist; und Anpassen (433) eines Modellparameters des Entrauschungsmodells auf der Grundlage des Unterscheidungsergebnisses, wobei das Erhalten des EEG-Signalentrauschungsmodells der EEG-Erfassungsvorrichtung durch Trainieren eines Entrauschungsmodells auf der Grundlage des erfassten EEG-Signals Folgendes umfasst:

Erhalten eines Entrauschungsmodells einer ersten Stufe (610) durch Durchführen eines Trainierens einer ersten Stufe an einem anfänglichen Entrauschungsmodell auf der Grundlage des erfassten EEG-Signals, das dem ersten analogen EEG-Signal entspricht;

Erhalten eines Entrauschungsmodells einer zweiten Stufe (620) durch Durchführen eines Trainierens einer zweiten Stufe an dem Entrauschungsmodell der ersten Stufe auf der Grundlage des erfassten EEG-Signals, das dem zweiten analogen EEG-Signal entspricht; und

Erhalten des EEG-Signalentrauschungsmodells der EEG-Erfassungsvorrichtung durch Durchführen eines Trainierens einer dritten Stufe (630) an dem Entrauschungsmodells der zweiten Stufe auf der Grundlage des erfassten EEG-Signals, das dem dritten analogen EEG-Signal entspricht.

2. Verfahren nach Anspruch 1, wobei das Erzeugen eines analogen EEG-Signals Folgendes umfasst:

Erhalten eines Erzeugungsmodells; und Erzeugen des zweiten analogen EEG-Signals auf der Grundlage des Erzeugungsmodells.

3. Verfahren nach Anspruch 2, wobei das Erhalten eines Erzeugungsmodells Folgendes umfasst: Erhalten des Erzeugungsmodell durch Trainieren eines generativen adversarialen Netzwerks (GAN), wobei das Erzeugungsmodell ein Generator in dem generativen adversarialen Netzwerk ist.

4. Verfahren nach Anspruch 1, wobei das Anpassen eines Modellparameters des Entrauschungsmodells auf der Grundlage des Unterscheidungsergebnisses Folgendes umfasst:

in der aktuellen Trainingsstufe,

Bestimmen einer ersten Gewichtung, die mit einer Signaldifferenz zwischen dem entrauschten EEG-Signal und dem entsprechenden sauberen EEG-Signal assoziiert ist, und einer zweiten Gewichtung, die mit dem Unterscheidungsergebnis assoziiert ist;

Bestimmen eines Zielverlustes auf der Grundlage der Signaldifferenz, des Unterscheidungsergebnisses, der ersten Gewichtung und der zweiten Gewichtung; und

Anpassen des Modellparameters des Entrauschungsmodells auf der Grundlage des Zielverlusts.

5. System, umfassend mindestens ein Speichermedium, das einen Trainingsanweisungssatz für ein EEG-Signalentrauschungsmodell (140) einer EEG-Erfassungsvorrichtung (120) speichert, und mindestens einen Prozessor in Kommunikation mit dem mindestens einen Speichermedium, wobei, wenn der Anweisungssatz durchgeführt wird, der mindestens eine Prozessor konfiguriert ist zum:

Erzeugen eines analogen EEG-Signals (110), wobei das analoge EEG-Signal ein erstes analoges EEG-Signal, das ein zugrunde liegendes Merkmal des analogen EEG-Signals widerspiegelt, ein zweites analoges EEG-Signal, das ein sauberes EEG-Signal simuliert, und ein drittes analoges EEG-Signal mit mindestens einem weiteren Rauschen einschließt, das dem sauberen EEG-Signal überlagert ist;

Erhalten eines erfassten EEG-Signals durch Erfassen des analogen EEG-Signals mittels der EEG-Erfassungsvorrichtung, wobei das erfasste EEG-Signal ein durch die EEG-Erfassungsvorrichtung verursachtes Rauschen enthält; und

Erhalten des EEG-Signalentrauschungsmodells der EEG-Erfassungsvorrichtung durch Trainieren eines Entrauschungsmodells auf der Grundlage des erfassten EEG-Signals, wobei das Trainieren des Entrauschungsmodells Folgendes umfasst:

Erhalten eines entrauschten EEG-Signals (150) durch Verarbeiten des erfassten EEG-Signals mittels des Entrauschungsmodells;

Erhalten eines Unterscheidungsergebnisses durch Verarbeiten des entrauschten EEG-Signals mittels eines Diskriminanzmodells, wobei das Unterscheidungsergebnis eine Wahrscheinlichkeit widerspiegelt, dass das Diskriminanzmodell bestimmt, dass das verarbeitete entrauschte EEG-Signal das saubere EEG-Signal ist; und

Anpassen eines Modellparameters des Entrauschungsmodells auf der Grundlage des Unterscheidungsergebnisses, wobei das Erhalten des EEG-Signalentrauschungsmodells der EEG-Erfassungsvorrichtung durch Trainieren eines Entrauschungsmodells auf der Grundlage des erfassten EEG-Signals Folgendes umfasst:

Erhalten eines Entrauschungsmodells einer ersten Stufe durch Durchführen eines Trainierens einer ersten Stufe an einem anfänglichen Entrauschungsmodell auf der Grundlage des erfassten EEG-Signals, das dem ersten analogen EEG-Signal entspricht;
Erhalten eines Entrauschungsmodells einer zweiten Stufe durch Durchführen eines Trainierens einer zweiten Stufe an dem Entrauschungsmodell der ersten Stufe auf der Grundlage des erfassten EEG-Signals, das dem zweiten analogen EEG-Signal entspricht; und
Erhalten des EEG-Signalentrauschungsmodells der EEG-Erfassungsvorrichtung durch Durchführen eines Trainierens einer dritten Stufe an dem Entrauschungsmodells der zweiten Stufe auf der Grundlage des erfassten EEG-Signals, das dem dritten analogen EEG-Signal entspricht.

6. System nach Anspruch 5, wobei der mindestens eine Prozessor weiter konfiguriert zum:

Erhalten eines Erzeugungsmodells; und
Erzeugen des zweiten analogen EEG-Signals auf der Grundlage des Erzeugungsmodells.

7. System nach Anspruch 6, wobei der mindestens eine Prozessor weiter konfiguriert ist zum:
Erhalten des Erzeugungsmodell durch Trainieren eines generativen adversarialen Netzwerks, wobei das Erzeugungsmodell ein Generator in dem generativen adversarialen Netzwerk ist.

8. Trainingsvorrichtung für ein EEG-Signalentrauschungsmodell (140) einer EEG-Erfassungsvorrichtung (120), einschließlich mindestens eines Speichermediums, das konfiguriert ist, um Computeranweisungen zu speichern, und mindestens eines Prozessors, der konfiguriert ist, um die Computeranweisungen durchzuführen, um das Verfahren zu implementieren zum:

Erzeugen eines analogen EEG-Signals (110), wobei das analoge EEG-Signal ein erstes analoges EEG-Signal, das ein zugrunde liegendes Merkmal des analogen EEG-Signals widerspiegelt, ein zweites analoges EEG-Signal, das ein sauberes EEG-Signal simuliert, und ein drittes analoges EEG-Signal mit mindestens einem weiteren Rauschen einschließt, das dem sauberen EEG-Signal überlagert ist
Erhalten eines erfassten EEG-Signals durch Erfassen des analogen EEG-Signals mittels der EEG-Erfassungsvorrichtung, wobei das erfasste EEG-Signal ein durch die EEG-Erfassungsvorrichtung verursachtes Rauschen enthält; und
Erhalten des EEG-Signalentrauschungsmodells der EEG-Erfassungsvorrichtung durch Trainieren eines Entrauschungsmodells auf der Grundlage des erfassten EEG-Signals, wobei das Trainieren des Entrauschungsmodells Folgendes umfasst:

Erhalten eines entrauschten EEG-Signals (150) durch Verarbeiten des erfassten EEG-Signals mittels des Entrauschungsmodells;
Erhalten eines Unterscheidungsergebnisses durch Verarbeiten des entrauschten EEG-Signals mittels eines Diskriminanzmodells, wobei das Unterscheidungsergebnis eine Wahrscheinlichkeit widerspiegelt, dass das Diskriminanzmodell bestimmt, dass das verarbeitete entrauschte EEG-Signal das saubere EEG-Signal ist; und
Anpassen eines Modellparameters des Entrauschungsmodells auf der Grundlage des Unterscheidungsergebnisses, wobei das Erhalten des EEG-Signalentrauschungsmodells der EEG-Erfassungsvorrichtung durch Trainieren eines Entrauschungsmodells auf der Grundlage des erfassten EEG-Signals Folgendes umfasst:

Erhalten eines Entrauschungsmodells einer ersten Stufe durch Durchführen eines Trainierens einer ersten Stufe an einem anfänglichen Entrauschungsmodell auf der Grundlage des erfassten EEG-Signals, das dem ersten analogen EEG-Signal entspricht;
Erhalten eines Entrauschungsmodells einer zweiten Stufe durch Durchführen eines Trainierens einer zweiten Stufe an dem Entrauschungsmodell der ersten Stufe auf der Grundlage des erfassten EEG-Signals, das dem zweiten analogen EEG-Signal entspricht; und
Erhalten des EEG-Signalentrauschungsmodells der EEG-Erfassungsvorrichtung durch Durchführen eines Trainierens einer dritten Stufe an dem Entrauschungsmodells der zweiten Stufe auf der Grundlage des erfassten EEG-Signals, das dem dritten analogen EEG-Signal entspricht.

9. Computerlesbares Speichermedium, das Computeranweisungen speichert, wobei ein Computer beim Lesen der Computeranweisungen ein Verfahren implementiert, das Folgendes umfasst:

Erzeugen eines analogen EEG-Signals (110), wobei das analoge EEG-Signal ein erstes analoges EEG-Signal, das ein zugrunde liegendes Merkmal des analogen EEG-Signals widerspiegelt, ein zweites analoges EEG-Signal, das ein sauberes EEG-Signal simuliert, und ein drittes analoges EEG-Signal mit mindestens einem weiteren Rauschen einschließt, das dem sauberen EEG-Signal überlagert ist

Erhalten eines erfassten EEG-Signals durch Erfassen des analogen EEG-Signals mittels der EEG-Erfassungsvorrichtung (120), wobei das erfasste EEG-Signal ein durch die EEG-Erfassungsvorrichtung verursachtes Rauschen enthält; und

Erhalten des EEG-Signalentrauschungsmodells (140) der EEG-Erfassungsvorrichtung durch Trainieren eines Entrauschungsmodells auf der Grundlage des erfassten EEG-Signals, wobei das Trainieren des Entrauschungsmodells Folgendes umfasst:

Erhalten eines entrauschten EEG-Signals (150) durch Verarbeiten des erfassten EEG-Signals mittels des Entrauschungsmodells;

Erhalten eines Unterscheidungsergebnisses durch Verarbeiten des entrauschten EEG-Signals mittels eines Diskriminanzmodells, wobei das Unterscheidungsergebnis eine Wahrscheinlichkeit widerspiegelt, dass das Diskriminanzmodell bestimmt, dass das verarbeitete entrauschte EEG-Signal das saubere EEG-Signal ist; und

Anpassen eines Modellparameters des Entrauschungsmodells auf der Grundlage des Unterscheidungsergebnisses, wobei das Erhalten des EEG-Signalentrauschungsmodells der EEG-Erfassungsvorrichtung durch Trainieren eines Entrauschungsmodells auf der Grundlage des erfassten EEG-Signals Folgendes umfasst:

Erhalten eines Entrauschungsmodells einer ersten Stufe durch Durchführen eines Trainierens einer ersten Stufe an einem anfänglichen Entrauschungsmodell auf der Grundlage des erfassten EEG-Signals, das dem ersten analogen EEG-Signal entspricht;

Erhalten eines Entrauschungsmodells einer zweiten Stufe durch Durchführen eines Trainierens einer zweiten Stufe an dem Entrauschungsmodell der ersten Stufe auf der Grundlage des erfassten EEG-Signals, das dem zweiten analogen EEG-Signal entspricht; und

Erhalten des EEG-Signalentrauschungsmodells der EEG-Erfassungsvorrichtung durch Durchführen eines Trainierens einer dritten Stufe an dem Entrauschungsmodells der zweiten Stufe auf der Grundlage des erfassten EEG-Signals, das dem dritten analogen EEG-Signal entspricht.

10. Verfahren nach Anspruch 1, wobei das erste analoge EEG-Signal ein sinusförmiges Signal einschließt, das das zugrunde liegende Merkmal des analogen EEG-Signals widerspiegelt.

11. Verfahren nach Anspruch 4, wobei,

die erste Gewichtung einen wesentlichen Grad der Signaldifferenz zur Modelloptimierung in der aktuellen Trainingsstufe angibt, und

die zweite Gewichtung einen wesentlichen Grad des Unterscheidungsergebnisses für die Modelloptimierung in der aktuellen Trainingsstufe angibt.

12. Verfahren nach Anspruch 11, weiter umfassend:

in der ersten Trainingsstufe, Einstellen der ersten Gewichtung, um größer zu sein als die zweite Gewichtung, damit sich die Entrauschungsmodelloptimierung stärker auf die Signaldifferenz und weniger auf das Unterscheidungsergebnis konzentriert; und

in der zweiten Stufe, Einstellen der ersten Gewichtung, um kleiner zu sein als die zweite Gewichtung, damit sich die Entrauschungsmodelloptimierung stärker auf das Unterscheidungsergebnis und weniger auf die Signaldifferenz konzentriert.

## Revendications

1. Procédé (400) mis en œuvre sur un dispositif informatique, le dispositif informatique incluant au moins un processeur et au moins un support de stockage stockant un ensemble d'instructions d'entraînement pour l'entraînement d'un modèle de débruitage de signal électroencéphalographique (EEG) d'un dispositif de collecte EEG, le procédé comprenant :

la génération (410) d'un signal EEG analogique, dans lequel le signal EEG analogique inclut un premier signal EEG analogique reflétant une caractéristique sous-jacente du signal EEG analogique, un deuxième signal EEG analogique simulant un signal EEG propre, et un troisième signal EEG analogique avec au moins un autre bruit superposé au signal EEG propre ;

l'obtention (420) d'un signal EEG collecté en collectant le signal EEG analogique à travers le dispositif de collecte EEG, le signal EEG collecté contenant un bruit causé par le dispositif de collecte EEG ; et

l'obtention (430) du modèle de débruitage de signal EEG du dispositif de collecte EEG en entraînant un modèle de débruitage basé sur le signal EEG collecté, l'entraînement du modèle de débruitage comprenant :

l'obtention (431) d'un signal EEG débruité en traitant le signal EEG collecté à travers le modèle de débruitage ;

l'obtention (432) d'un résultat de discrimination en traitant le signal EEG débruité à travers un modèle discriminant, le résultat de discrimination reflétant une probabilité que le modèle discriminant détermine que le signal EEG débruité traité est le signal EEG propre ; et

l'ajustement (433) d'un paramètre de modèle du modèle de débruitage sur la base du résultat de discrimination, dans lequel l'obtention du modèle de débruitage de signal EEG du dispositif de collecte EEG en entraînant un modèle de débruitage basé sur le signal EEG collecté comprend :

l'obtention d'un modèle de débruitage de première phase (610) en effectuant un entraînement de première phase sur un modèle de débruitage initial basé sur le signal EEG collecté correspondant au premier signal EEG analogique ;

l'obtention d'un modèle de débruitage de deuxième phase (620) en effectuant un entraînement de deuxième phase sur le modèle de débruitage de première phase basé sur le signal EEG collecté correspondant au deuxième signal EEG analogique ; et

l'obtention du modèle de débruitage de signal EEG du dispositif de collecte EEG en effectuant un entraînement de troisième phase (630) sur le modèle de débruitage de deuxième phase basé sur le signal EEG collecté correspondant au troisième signal EEG analogique.

2. Procédé selon la revendication 1, la génération d'un signal EEG analogique comprenant :

l'obtention d'un modèle de génération ; et
la génération du deuxième signal EEG analogique basé sur le modèle de génération.

3. Procédé selon la revendication 2, l'obtention d'un modèle de génération comprenant :
l'obtention du modèle de génération en entraînant un réseau antagoniste génératif (GAN), dans lequel le modèle de génération est un générateur dans le réseau antagoniste génératif.

4. Procédé selon la revendication 1, l'ajustement d'un paramètre de modèle du modèle de débruitage sur la base du résultat de discrimination comprenant :

dans la phase d'entraînement courant,
la détermination d'un premier poids associé à une différence de signal entre le signal EEG débruité et le signal EEG propre correspondant et d'un second poids associé au résultat de discrimination ;

la détermination d'une perte cible sur la base de la différence de signal, du résultat de discrimination, du premier poids et du second poids ; et l'ajustement du paramètre de modèle du modèle de débruitage sur la base de la perte cible.

5. Système comprenant au moins un support de stockage stockant un ensemble d'instructions d'entraînement pour un modèle de débruitage de signal EEG (140) d'un dispositif de collecte EEG (120) et au moins un processeur en communication avec l'au moins un support de stockage, dans lequel, lors de la mise en œuvre de l'ensemble d'instructions, l'au moins un processeur est configuré pour :

générer un signal EEG analogique (110),
dans lequel le signal EEG analogique inclut un premier signal EEG analogique reflétant une caractéristique sous-jacente du signal EEG analogique, un deuxième signal EEG analogique simulant un signal EEG propre et un troisième signal EEG analogique avec au moins un autre bruit superposé au signal EEG propre ;

obtenir un signal EEG collecté en collectant le signal EEG analogique à travers le dispositif de collecte EEG, le signal EEG collecté contenant un bruit causé par le dispositif de collecte EEG ; et

obtenir le modèle de débruitage de signal EEG du dispositif de collecte EEG en entraînant un modèle de débruitage basé sur le signal EEG collecté, l'entraînement du modèle de débruitage comprenant :

l'obtention d'un signal EEG débruité (150) en traitant le signal EEG collecté à travers le modèle de débruitage ;

l'obtention d'un résultat de discrimination en traitant le signal EEG débruité à travers un modèle discriminant, le résultat de discrimination reflétant une probabilité que le modèle discriminant détermine que le signal EEG débruité traité est le signal EEG propre ;

l'ajustement d'un paramètre de modèle du modèle de débruitage sur la base du résultat de discrimination, dans lequel l'obtention du modèle de débruitage de signal EEG du dispositif de collecte EEG en entraînant un modèle de débruitage basé sur le signal EEG collecté comprend :

l'obtention d'un modèle de débruitage de première phase en effectuant un entraînement de première phase sur un modèle de débruitage initial basé sur le signal EEG collecté correspondant au premier signal EEG analogique ;

l'obtention d'un modèle de débruitage de deuxième phase en effectuant un entraînement de deuxième phase sur le modèle de débruitage de première phase basé sur le signal EEG collecté correspondant au deuxième signal EEG analogique ; et

l'obtention du modèle de débruitage de signal EEG du dispositif de collecte EEG en effectuant un entraînement de troisième phase sur le modèle de débruitage de deuxième phase basé sur le signal EEG collecté correspondant au troisième signal EEG analogique.

6. Système selon la revendication 5, dans lequel l'au moins un processeur est en outre configuré pour :

obtenir un modèle de génération ; et
générer le deuxième signal EEG analogique basé sur le modèle de génération.

7. Système selon la revendication 6, dans lequel l'au moins un processeur est en outre configuré pour :
obtenir le modèle de génération en entraînant un réseau antagoniste génératif, dans lequel le modèle de génération est un générateur dans le réseau antagoniste génératif.

8. Dispositif d'entraînement pour un modèle de débruitage de signal EEG (140) d'un dispositif de collecte EEG (120), incluant au moins un support de stockage configuré pour stocker des instructions informatiques et au moins un processeur configuré pour effectuer les instructions informatiques afin de mettre en œuvre le procédé de :

génération d'un signal EEG analogique (110), dans lequel le signal EEG analogique inclut un premier signal EEG analogique reflétant une caractéristique sous-jacente du signal EEG analogique, un deuxième signal EEG analogique simulant un signal EEG propre, et un troisième signal EEG analogique avec au moins un autre bruit superposé au signal EEG propre
obtention d'un signal EEG collecté par la collecte du signal EEG analogique à travers le dispositif de collecte EEG, le signal EEG collecté contenant un bruit causé par le dispositif de collecte EEG ; et
obtention du modèle de débruitage de signal EEG du dispositif de collecte EEG en entraînant un modèle de débruitage basé sur le signal EEG collecté, l'entraînement du modèle de débruitage comprenant :

l'obtention d'un signal EEG débruité (150) en traitant le signal EEG collecté à travers le modèle de débruitage ;

l'obtention d'un résultat de discrimination en traitant le signal EEG débruité à travers un modèle discriminant, le résultat de discrimination reflétant une probabilité que le modèle discriminant détermine que le signal EEG débruité traité est le signal EEG propre ; et

l'ajustement d'un paramètre de modèle du modèle de débruitage sur la base du résultat de discrimination, dans lequel l'obtention du modèle de débruitage de signal EEG du dispositif de collecte EEG en entraînant un modèle de débruitage basé sur le signal EEG collecté comprend :

l'obtention d'un modèle de débruitage de première phase en effectuant un entraînement de première phase sur un modèle de débruitage initial basé sur le signal EEG collecté correspondant au premier signal EEG analogique ;

l'obtention d'un modèle de débruitage de deuxième phase en effectuant un entraînement de deuxième phase sur le modèle de débruitage de première phase basé sur le signal EEG collecté correspondant au deuxième signal EEG analogique ; et

l'obtention du modèle de débruitage de signal EEG du dispositif de collecte EEG en effectuant un entraînement de troisième phase sur le modèle de débruitage de deuxième phase basé sur le signal EEG collecté correspondant au troisième signal EEG analogique.

9. Support de stockage lisible par ordinateur stockant des instructions informatiques, dans lequel, lors de la lecture des instructions informatiques, un ordinateur met en œuvre un procédé comprenant :

la génération d'un signal EEG analogique (110), dans lequel le signal EEG analogique inclut un premier signal EEG analogique reflétant une caractéristique sous-jacente du signal EEG analogique, un deuxième signal EEG analogique simulant un signal EEG propre, et un troisième signal EEG analogique avec au moins un autre bruit superposé au signal EEG propre

l'obtention d'un signal EEG collecté par collecte du signal EEG analogique à travers le dispositif de collecte EEG (120), le signal EEG collecté contenant un bruit causé par le dispositif de collecte EEG ; et

l'obtention du modèle de débruitage de signal EEG (140) du dispositif de collecte EEG en entraînant un modèle de débruitage basé sur le signal EEG collecté, l'entraînement du modèle de débruitage comprenant :

l'obtention d'un signal EEG débruité (150) en traitant le signal EEG collecté à travers le modèle de débruitage ;

l'obtention d'un résultat de discrimination en traitant le signal EEG débruité à travers un modèle discriminant, le résultat de discrimination reflétant une probabilité que le modèle discriminant détermine que le signal EEG débruité traité est le signal EEG propre ; et

l'ajustement d'un paramètre de modèle du modèle de débruitage sur la base du résultat de discrimination, dans lequel l'obtention du modèle de débruitage de signal EEG du dispositif de collecte EEG en entraînant un modèle de débruitage basé sur le signal EEG collecté comprend :

l'obtention d'un modèle de débruitage de première phase en effectuant un entraînement de première phase sur un modèle de débruitage initial basé sur le signal EEG collecté correspondant au premier signal EEG analogique ;

l'obtention d'un modèle de débruitage de deuxième phase en effectuant un entraînement de deuxième phase sur le modèle de débruitage de première phase basé sur le signal EEG collecté correspondant au deuxième signal EEG analogique ; et

l'obtention du modèle de débruitage de signal EEG du dispositif de collecte EEG en effectuant un entraînement de troisième phase sur le modèle de débruitage de deuxième phase basé sur le signal EEG collecté correspondant au troisième signal EEG analogique.

10. Procédé selon la revendication 1, dans lequel le premier signal EEG analogique inclut un signal sinusoïdal reflétant la caractéristique sous-jacente du signal EEG analogique.

11. Procédé selon la revendication 4, dans lequel

le premier poids indique un degré important de différence de signal pour une optimisation de modèle dans la phase d'entraînement courante, et

le second poids indique un degré important du résultat de discrimination pour l'optimisation de modèle dans la phase d'entraînement courante.

12. Procédé selon la revendication 11, comprenant en outre :

dans la première phase d'entraînement, une fixation du premier poids sur une valeur supérieure au second poids afin que l'optimisation de modèle de débruitage se concentre davantage sur la différence de signal et moins sur le résultat de discrimination ; et

dans la deuxième phase, une fixation du premier poids sur une valeur inférieure au second poids afin que l'optimisation de modèle de débruitage se concentre davantage sur le résultat de discrimination et moins sur la différence de signal.

100

FIG. 1

**FIG. 2**

**300**

**400**

FIG. 4

**430**

```
┌─────────────────────────────────────────────────┐  431
│                                                   │
│  Obtaining a denoised EEG signal by processing    │
│  the collected EEG signal through the             │
│  denoising model                                  │
│                                                   │
└─────────────────────────────────────────────────┘
                        │
                        ▼
┌─────────────────────────────────────────────────┐  432
│                                                   │
│  Obtaining a discrimination result by processing  │
│  the denoised EEG signal through a discriminant   │
│  model, the discrimination result reflecting a    │
│  probability that the discriminant model          │
│  determines that the processed denoised EEG       │
│  signal is a clean EEG signal                     │
└─────────────────────────────────────────────────┘
                        │
                        ▼
┌─────────────────────────────────────────────────┐  433
│                                                   │
│  Adjusting a model parameter of the denoising     │
│  model based on the discrimination result         │
│                                                   │
└─────────────────────────────────────────────────┘
```

**FIG. 5**

**FIG. 6**

**700**

Determining a first weight associated with a signal difference between a denoised EEG signal and a corresponding clean EEG signal and a second weight associated with a discrimination result

Determining a target loss based on the signal difference, the discrimination result, the first weight, and the second weight

Adjusting a model parameter of a denoising model based on the target loss

**FIG. 7**

**800** 810

Generation module

820

Collection module

830

Training module

**FIG. 8**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202111503309
- CN 113349800 A

- WO 2020128134 A1